# EUROPÄISCHE PATENTSCHRIFT

(11) **EP 2 593 042 B1**
(45) Veröffentlichungstag und Bekanntmachung des Hinweises auf die Patenterteilung: **25.05.2016**
(21) Anmeldenummer: 11735634.5
(22) Anmeldetag: 13.07.2011
(51) Int. Cl.: A61F 2/42, A61F 2/30

(54) **FINGERGELENKPROTHESE**
FINGER JOINT PROSTHESIS
PROTHÈSE D'ARTICULATION DIGITALE

(30) Priorität: 14.07.2010 DE 102010031349
(43) Veröffentlichungstag der Anmeldung: 22.05.2013
(73) Patentinhaber: Waldemar Link GmbH & Co. KG, 22339 Hamburg (DE)
(72) Erfinder: LINK, Helmut D., 22397 Hamburg (DE)
(74) Vertreter: Lippert, Stachow & Partner
(86) Internationale Anmeldenummer: PCT/EP2011/062000
(87) Internationale Veröffentlichungsnummer: WO 2012/007528

(56) Entgegenhaltungen:
- EP-A1- 1 096 906
- WO-A1-90/11739
- DE-A1-102007 008 406

## Beschreibung

Die Erfindung betrifft eine Fingergelenkprothese mit einem Gelenkkörper, der ein erstes und ein zweites Gelenkelement aufweist. Die Gelenkelemente sind schwenkbar um eine Drehachse miteinander verbunden und jedes Gelenkelement umfasst eine längliche Verankerungsleiste zur Verwirklichung einer sekundären Befestigung an einem proximalen und/oder distalen Fingerglied. Zusätzlich können an den Verankerungsleisten eine oder mehrere Ösen mit Öffnungen zur Aufnahme von Fixierungsschrauben zur Realisierung einer primären Fixierung ausgebildet. Diese Fixierschrauben erfüllen die Primärfixierung zur Fixierung der Prothese in der Sollposition an den Knochen. Die Fingergelenkprothese wächst sodann im Rahmen der biologischen- oder Sekundär-Fixierung im Knochen an. Die Sekundär-Fixierung erfolgt dann über die Oberfläche der Fingergelenkprothese.

Funktionsstörungen von Fingermittelgelenken können zum einen aufgrund einer vererbten degenerativen Erkrankung, der Gelenkarthrose, entstehen. In diesem Fall führt die Erkrankung zum "Verschleiß" des Gelenkknorpels und in der Folge zu entsprechenden Fehlbelastungen mit kompletten Veränderungen der Gelenkflächen, die eine schmerzhafte Einschränkung oder sogar Aufhebung der Gelenkfunktion zur Folge haben. Zum anderen können Funktionsstörungen der Fingermittelgelenke auch aufgrund einer Verletzung, z.B. durch Luxation des Gelenkes oder durch einen Gelenkbruch entstehen. Eine primäre Behandlung eines Gelenkbruches führt in der Regel zu einer so genannten "posttraumatischen Arthrose" des Gelenkes, was ebenfalls zu einer schmerzhaften Funktionseinschränkung oder Funktionsaufhebung des Fingermittelgelenkes führt.

Ein Weg zur Beseitigung der oben genannten Funktionsstörungen ist die Versteifung des Gelenkes in eine funktionsgünstige Stellung. Dieser Weg führt zwar zur Schmerzfreiheit, bedeutet aber die vollständige Funktionsunfähigkeit des Gelenkes.

Für die Implantation von älteren Fingergelenkprothesen musste für einen ulnaren Zugang das innere Seitenband an dem Fingerglied abgelöst werden. Das Gelenk wurde dann seitlich luxiert und ein Teil der Palmarplatte abgelöst. Anschließend wurden der Kopf des ersten Fingergliedes und die Basis des zweiten Fingergliedes so reseziert, dass zwischen den beiden Fingergliedern ein vorher abgestimmter Abstand entstand. Daraufhin wurde in jedes Fingerglied ein sich entlang seiner Mittelachse erstreckender rechteckiger Raum geraspelt. In diesen rechteckigen Raum wurde eine Schaftführung einzementiert. Anschließend wurden die Gelenkelemente einzeln mit ihren Verankerungsleisten in die Schaftführung eingesetzt, die Fingerglieder zurückgebogen und das erste und das zweite Gelenkelement durch Einsetzen einer Achse in die fluchtenden Öffnungen gelenkig miteinander verbunden.

Da bei der Implantation der bekannten Fingergelenkprothesen das Fingermittelgelenk seitlich luxiert werden musste, wurden der Strecksehnenapparat, die beiden Beugesehnen sowie die Seitenbänder des Gelenkes irritiert, was zu einer späteren Beeinträchtigung der Funktionsfähigkeit führte. Darüber hinaus wurde eine große Menge an Knochensubstanz für die Implantation der Fingergelenkprothese geopfert, da eine Schaftführung in Richtung der Mittelachse der Fingergelenke eingeführt werden musste. Die DE 690 02159 offenbart eine solche Prothese, bei welcher gemäß Beschreibung ein reduzierter Verlust an Knochensubstanz bei Implantation zu verzeichnen ist.

Eine deutlich verbesserte Fingergelenkprothese ist aus der EP 1 096 906 des Erfinders Christoph Ranft bekannt. Diese ermöglicht ein neuartiges und verbessertes Verfahren unter Vermeidung der vorgenannten Nachteile. Bei dem in der EP 1 096 906 beschriebenen Verfahren kann die Fingergelenkprothese im zusammengebauten Zustand radial in eine vorgefertigte Bohrung im Fingergelenk implantiert werden, was eine deutliche Vereinfachung des Einbaus darstellt und zu geringen Verletzungen, insbesondere der Sehnen des Fingers führt.

Andererseits ist diese Fingergelenkprothese zu filigran aufgebaut und weist eine teilweise mangelnde Festigkeit und Haltbarkeit auf. In der DE 10 2007 008 406 ist eine radial implantierbare Fingergelenkprothese offenbart, die eine höhere Stabilität aufweist, gleichzeitig aber weiterhin einfach mit geringer Verletzung des Patienten implantierbar ist. Diese Aufgabe wird gemäß DE 10 2007 008 406 dadurch gelöst, dass das erste Gelenkelement einen Außenhohlkörper mit einer Einsatzöffnung aufweist, dass das zweite Gelenkelement einen Innenhohlkörper aufweist, dass der Innenhohlkörper in Einbaulage über die Einsatzöffnung relativ in den Außenhohlkörper einsetzbar ist, und dass eine Achse in Einbaulage zur drehbeweglichen Verbindung mit dem Außenhohlkörper in den Innenhohlkörper einsetzbar ist.

Obgleich die in der DE 10 2007 008 406 offenbarte Prothese eine gegenüber dem Stand der Technik erheblich verbesserte Stabilität aufweist, bestehen gleichwohl gewisse Schwächen bei der Verankerung, besonders bei Belastungen aus distalem Zug. Dies kann zur Lockerung der Fingergelenkprothese führen, insbesondere auf der distalen Verankerungsleiste. Dadurch kann das Anwachsen der Prothese in einer unerwünschten Lage erfolgen. Ferner kann ein Anwachsen (Sekundärfixierung) verzögert oder ganz ausbleiben.

Der Erfindung liegt mithin die Aufgabe zugrunde, eine eingangs genannte Fingergelenkprothese derart weiter zu entwickeln, dass die vorgenannten Nachteile, insbesondere das Problem der distalen Lockerung, zumindest teilweise vermieden werden und folglich eine Fingergelenkprothese mit einer verbesserten Primär- und Sekundärfixierung bereitzustellen.

Diese Aufgabe wird erfindungsgemäß dadurch gelöst, dass von der Drehachse distal an der Verankerungsleiste von zumindest einem Gelenkelement ein endseitiges Befestigungselement vorgesehen ist, welches medial in Bezug auf die horizontale Mittelebene der Verankungsleiste angeordnet ist.

Die Mittelebene verliefe dabei parallel zu der durch die flächige Verankerungsleiste aufgespannten Ebene. Der Mittelpunkt des endseitigen Befestigungselements liegt dann also auf der horizontalen Mittelebene einer Verankerungsleiste. Das bedeutet, dass das endseitige Befestigungselement in Bezug auf die Mittelebene der Verankerungsleiste medial und explizit nicht lateral angeordnet ist. Vorteilhafterweise könnten derart laterale, proximale Befestigungselemente, wie beispielsweise Ösen, ergänzt und/oder ganz oder teilweise ersetzt werden.

Die Ausbildung eines solchen endseitigen Befestigungselements bietet gegenüber der in der DE 10 2007 008 406 dargelegten Ausführungsform den Vorteil, dass die Prothese bei den erfindungsgemäßen Ausführungen des endseitigen Befestigungselements eine stärkere Verankerung, auch in Bezug auf Belastungen aus distalem Zug und insbesondere ein verbessertes Anwachsen an den Knochen ermöglicht. Die Fixierung kann zudem medial zu der Fingermittelachse erfolgen. Somit ist es möglich, der vorgenannten Problematik beim Stand der Technik besonders gut entgegenzuwirken, insbesondere in Bezug auf ungewollte Hebelwirkungen. Durch die Erfindung kann eine verbesserte Primär- und Sekundärfixierung realisiert werden. Es kann so eine Lockerung der Fingergelenkprothese, insbesondere auf der distalen Verankerungsleiste, verhindert und deren Folgen vermieden werden. Diese Wirkung kommt unter anderem dadurch zustande, dass ein besserer Eingriff der Befestigungselemente in die mediale Korticalis sowie in den spongiösen Bereich ermöglicht wird. Ungünstige Hebelwirkungen werden durch die endseitige Anordnung des Befestigungselements vermieden und die Oberfläche der Prothese wird gleichzeitig vergrößert. Es hat sich bei der Entwicklung der Erfindung herausgestellt, dass die Probleme beim Stand der Technik u.a. bedingt sind durch die Fixierung der Fingergelenkprothese am radialen Ansatz des Fingers außerhalb der Fingermittelachse. Diese Problematik wird durch die Erfindung vermieden, bzw. reduziert.

Das endseitige Befestigungselement kann auf verschiedenste Weise zur Befestigung der Fingergelenkprothese am Finger ausgebildet sein, beispielsweise durch eine Ausführung des Befestigungselements als endseitige Befestigungsöse. Dabei kann die endseitige Befestigungsöse ebenfalls medial in Bezug auf die horizontale Mittelebene der Verankerungsleiste angeordnet sein. Diese im Verlauf der Verankerungsleiste, vorzugsweise einstückig angeordnete Öse ist ausgebildet für die Aufnahme von Zugkräften. Diese spezielle Anordnung bietet gegenüber der bekannten Ausführung den Vorteil, dass keine Ausnehmung in der jeweiligen Verankerungsleiste vorgesehen werden muss, um auch schräges Einschrauben in den Knochen zu ermöglichen. Die somit bewerkstelligte Flächenvergrößerung der Verankerungsleiste ermöglicht ebenfalls ein verbessertes Anwachsen an den Knochen und eine stärkere Verankerung. Da keine Ausnehmungen in der Fläche der Verankerungsleiste ausgebildet sein müssen, kann die Gestalt der Verankerungsleiste filigraner oder bessere angepasst an die Geometrie die Fingers gestaltet werden; so kann z.B. auf einem vorderseitigen Vorsprung im Gelenkbereich zur Kompensation der Kerbwirkung einer Ausnehmung verzichtet werden. Es wird außerdem ein besserer Eingriff der Verschraubung in den spongiösen Bereich und in die mediale Korticalis erreicht. Durch die endseitige Anordnung der Verschraubung werden ungünstige Hebelkräfte reduziert und ein besserer Halt gegen seitliches Ausbrechen erzielt.

Das endseitige Befestigungselement kann z.B. auch aus zwei benachbarten, endseitigen Ösen bestehen. Diese zusätzliche Fixierung verstärkt die Verankerung der Prothese zusätzlich und ermöglicht ein besseres Einwachsen.

Als besonders zweckmäßig hat sich die Ausführung des endseitigen Befestigungselements als Zylinder erwiesen, der entlang der Breite der Verankerungsleiste verläuft. Ein solcher Zylinder kann im Knochen "verkeilt" werden und bewerkstelligt so eine stärkere Verankerung gegen Belastungen, auch aus distalem Zug. Gleichzeitig ermöglicht die Flächenvergrößerung durch den Zylinder ein verbessertes Anwachsen an den Knochen. Die endseitige Anordnung des Zylinders reduziert ungünstige Hebelwirkungen und greift in den spongiösen Bereich und in die mediale Korticalis ein. Die zylindrische Form ist auch deshalb vorteilhaft, da die entsprechende Aussparung im Knochen durch bloßes Bohren anstatt aufwändiges Fräsen gefertigt werden kann. Besonders bevorzugt ist der Zylinder auf der horizontalen Mittelebene der Verankerungsleiste zudem medial angeordnet.

Die Oberfläche des endseitigen Befestigungselements und/oder der gesamten Fingergelenkprothese kann zusätzlich rau ausgebildet sein, um ein verbessertes Anwachsen zu unterstützen.

Es liegt natürlich auch im Rahmen der Erfindung, die beiden eben beschriebenen und weitere Ausführungsformen des endseitigen Befestigungselements zu kombinieren.

Denn die Geometrie des endseitigen Befestigungselements erschöpft sich natürlich nicht in der Ausbildung als Öse oder Zylinder, vielmehr sind weitere Gestaltungen denkbar, die derart ausgebildet sind, um Zugkräfte noch besser aufzunehmen sowie ein verbessertes Anwachsen und eine verbesserte Verankerung zu ermöglichen.

Zur Überbrückung von größeren Gewebeschädigungen kann ein Interponat vorgesehen sein. Ein solches Interponat kann besonders einfach als "Platzhalter" in Form eines polierten Zylinders ausgebildet sein. Die Abmaße des Zylinders entsprechen in Durchmesser und Länge dem bestehenden Fingergelenk oder einem sonstigen Gelenk. Auch um Verletzungen zu vermeiden, sollte der Zylinder nach Implantation nicht medial oder lateral überstehen. Ferner sollte der Außenbereich des Zylinders abgerundet sein. In diesem Sinne ist es ebenfalls zweckmäßig, dass alle Kanten an den jeweiligen Knochen harmonisch anpassbar sind. Cobalt-Chrom-Molybdän-Legierungen (CoCrMo) sowie Titan mit einer Titan-Niob-Beschichtung sind als Materialien für das Interponat besonders geeignet. Die beschriebenen Charakteristika des Interponats lassen sich analog auf alle denkbaren Ausbildungen des endseitigen Befestigungselements und weitere Bauteile der erfindungsgemäßen Fingergelenkprothese, soweit zweckmäßig, übertragen.

Auch die Verwendung eines Scharniers ist möglich, bei welchem das erste Gelenkelement einen Außenhohlkörper mit einer Einsatzöffnung aufweist, dass das zweite Gelenkelement einen Innenhohlkörper aufweist, dass der Innenhohlkörper in Einbaulage über die Einsatzöffnung relativ in den Außenhohlkörper einsetzbar ist, und dass eine Achse in Einbaulage zur drehbeweglichen Verbindung mit dem Außenhohlkörper in den Innenhohlkörper einsetzbar ist. Sowohl der Außenhohlkörper als auch der Innenhohlkörper sind jeweils mit einer Außenumfangsfläche und einer Innenumfangsfläche ausgebildet. Die Außenumfangsfläche des Innenhohlkörpers ist geringfügig kleiner ausgebildet als die Innenumfangsfläche des Außenhohlkörpers, damit der Innenhohlkörper in Einbaulage relativbeweglich in den Außenhohlkörper einsetzbar ist. Sowohl der Außenhohlkörper als auch der Innenhohlkörper sind rotationssymmetrisch ausgebildet und weisen dieselbe Geometrie auf, um eine störungsfreie Rotation zu gewährleisten.

In der besonders bevorzugten Ausführungsform des Scharniers sind der Außenhohlkörper und der Innenhohlkörper als Hohlzylinder ausgebildet.

Als besonders vorteilhaft hat sich herausgestellt, den Außenhohlkörper und den Innenhohlkörper aus Metall zu fertigen, da Metall eine hohe Stabilität und Dauerfestigkeit für den Einsatz in vivo aufweist. Besonders gute Gleit- und Reibeigenschaften weist dabei Titan auf, wobei besonders bevorzugt Titannitrit eingesetzt wird, welches biokompatibel ist und ein besonders gutes Abriebverhältnis hat. Dasselbe trifft natürlich auch auf die gesamte erfindungsgemäße Fingergelenkprothese zu.

Wenn die Hohlkörper aus Metall ausgebildet sind, kann die Achse aus Kunststoff ausgebildet sein, da diese eine besonders gute Materialpaarung mit Hohlkörpern bildet. Besonders bevorzugt ist die Verwendung von Polyethylen (PE). Es liegt jedoch im Rahmen der Erfindung, auch die Achse aus einem Metall auszubilden.

Zur Reduzierung der Reibkräfte zwischen den Stirnseiten der Innenhohlkörper und dem Außenhohlkörper können zwischen den Stirnflächen des Innenhohlkörpers und des Außenhohlkörpers Kunststoffscheiben eingesetzt werden, wobei diese vorzugsweise ebenfalls aus Polyethylen (PE) bestehen.

Die erfindungsgemäße Fingergelenkprothese ist vorzugsweise für ein proximales Interphalangealgelenk (PIP-Gelenk) verwendbar und kann die an diesem Gelenk auftretenden Kräfte problemlos aufnehmen. Zur Realisierung der gewünschten Kräfteaufnahme ist der Außendurchmesser des Außenhohlkörpers etwa 1,5 bis 2 Mal so groß wie der Außendurchmesser der Achse. Bei einem PIP-Gelenk weist der Außenhohlkörper vorzugsweise einen Außendurchmesser von 12 mm und die Achse einen Außendurchmesser von 6 mm auf. Die Verwendung als Ersatz für ein distales Interphalangealgelenk (DIP-Gelenk) oder gegebenenfalls auch ein Metacarpophalangealgelenk (MCP-Gelenk) liegt jedoch auch im Rahmen der Erfindung. Prinzipiell ist der erfindungsgemäße Aufbau auch für andere Gelenke bei entsprechend anderer Dimensionierung, aber unter Beibehaltung der zuvor beschriebenen vorteilhaften Größenverhältnisse realisierbar.

Anschlagmittel können zwischen dem Außen- und dem Innenhohlkörper ausgebildet sein, welche den Drehwinkel auf ein vorgegebenes Maß einschränken. Bei der besonders bevorzugten Ausführungsform sind die Anschlagmittel als Absätze an der äußeren Umfangsfläche des Innenhohlkörpers ausgebildet, die in Einbaulage gegen die Ränder der Einsatzöffnung des Außenhohlkörpers anschlagen. Vorzugsweise beträgt der Drehwinkel 0 bis 135 Grad, besonders bevorzugt 0 bis 90 Grad.

Es können ferner mehrere zapfenartige Ausnehmungen an der Verankerungsleiste vorgesehen sein. Diese Ausnehmungen sind vorzugsweise in Bezug auf laterale, proximale Befestigungsösen auf der Verankerungsleiste derart angeordnet, um auch ein schräges Einschrauben der Fixierungsschrauben zu ermöglichen. Da die vorgenannten Befestigungsösen an der Verankerungsleiste zwischen Drehachse und dem abgewandten Ende angeordnet sind, würden die Fixierungsschrauben beim Einschrauben ohne Ausnehmungen mit der Verankerungsleiste kollidieren. Um dies zu verhindern, können zudem Abschrägungen an der Verankerungsleiste ausgebildet sein. Die Befestigungsösen sind vorzugsweise leicht zur Vertikalen geneigt, so dass zwischen der Öse und der Fläche der Ebene der Verankerungsleiste ein spitzer Winkel eingeschlossen ist.

Während beim Stand der Technik auf der Vorder- und/oder Rückseite der Verankerungsleiste derartige Ausnehmungen nötig waren, da nur laterale Befestigungsösen vorgesehen sind, kann durch das erfindungsgemäße endseitige Befestigungselement auf Ausnehmungen ganz oder teilweise verzichtet werden. Dieses vergrößert in der oben beschriebenen Weise die Oberfläche der Verankerungsleiste, erhöht den Gestaltungsspielraum bei der Ausbildung der Verankerungsleiste, bewerkstelligt eine stärkere Verankerung und ermöglicht ein verbessertes Anwachsen an den Knochen.

Zur besseren Primärfixierung können an den Verankerungsleisten eine oder mehrere Rippen ausgebildet sein, welche sich vorzugsweise senkrecht zu der Fingermittelachse auf der Oberund/oder Unterseite der Verankerungsleisten erstrecken, um ein radiales Einschieben der Fingergelenkprothese in den Knochen zu ermöglichen. Die Rippen können im Querschnitt vieleckig und/oder abgerundet ausgebildet sein. Bevorzugte Querschnitte sind beispielsweise dreieckig, rechteckig sowie als Halbkreis ausgebildet. Die Rippen können an einer flächigen Seite oder auch an beiden Seiten einer Verankerungsleiste vorgesehen sein. Die durch die Rippen bewerkstelligte Oberflächenvergrößerung der Verankerungsleiste unterbindet das Versetzen, verbessert das Anwachen und ermöglicht so eine Verbesserung der Fixierung der Prothese erreicht. Vorzugsweise verjüngen diese Rippen zu der Seite der Verankerungsleiste hin, welche in den spongiösen Bereich eingreift. Damit wird das radiale Einschieben der Fingergelenkprothese beim Einbau in den Fingerknochen erleichtert.

Die Befestigungselemente der Fingergelenksprothese können auch ausschließlich distale endseitige Befestigungselemente umfassen, insbesondere die endseitigen Befestigungsösen. Ausnehmungen zum schrägen Verschrauben müssen bei dieser Ausbildung nicht vorgesehen werden, wodurch die Stabilität der Verankerungsleisten wegen der fehlenden Kerbwirkung im Vergleich zu den vorgenannten Ausführungsformen erheblich verbessert wird. Auch diese Ausbildung verbessert das Anwachsen, weil die Oberfläche der Verankerungsleiten vergrößert ist.

Ferner kann das erfindungsgemäße endseitige Befestigungselement gemäß einer Sonderform als flügelartige, horizontale Verbreiterung der distalen Verankerungsleiste ausgebildet sein. Die Verbreiterung ist vorzugsweise senkrecht zur Fingergelenksachse ausgebildet. Das horizontale flügelartige Befestigungselement erstreckt sich vorzugsweise vom distalen Ende der Verankerungsleiste bis zu einer Ausnehmung auf derselben Verankerungsleiste. Das in den spongiösen Knochen eingreifende Ende der flügelartigen Verbreiterung ist vorzugsweise mit einer Verjüngung ausgebildet. Auf diese Weise wird das Einführen der Fingergelenkprothese beim Einbau erleichtert. Die Verjüngung kann beispielsweise als Abrundung ausgebildet sein. Dieses hat zudem den Vorteil, dass die angepasste Aussparung im Kochen dann keine Ecken o.ä. aufweist, welche in bekannter Weise einen Riss bzw. Bruch im Knochen begünstigen. Dadurch kann dass Anwachsen und die Belastbarkeit der Fingergelenksprothese verbessert werden. Ferne kann die besagte Aussparung mit einer Kreissäge oder Ähnlichem, besonders einfach bewerkstelligt werden. Aufwendiges Fräsen kann vermieden werden.

Durch das flügelartige endseitige Befestigungselement, das sich in Einbaulage mindestens über die Fingergelenksmittelachse erstreckt, vorzugsweise etwa bis zur Länge der Achse des Gelenkkörpers, können ungünstige Hebelwirkungen in der Verankerungsleiste reduziert werden. Es ist so möglich, Brüche bedingt durch Materialermüdung der Fingergelenksprothese zu vermeiden, welche insbesondere im Bereich der Ausnehmungen auftreten können.

Bisher wurde versucht, durch Verbreiterung der Fläche der Verankerungsleiste Brüchen entgegenzuwirken. Diese Verbreitung war zumeist so an der Verankerungsleiste ausgebildet, dass diese in Einbaulage als nach vorne ragender Vorsprung oder "Höcker" über die jeweiligen Fingerknochen herausragten, auch um die Fingergelenkprothese radial in den Knochen einschieben zu können. Durch die flügelartige Verbreitung zur biomechanischen Fingermittelachse hin sind derartige Verbreiterungen der Fläche nicht notwendig zur Erzielung der erforderlichen Stabilität. Die Ausgestaltung der Verankerungsleisten kann nun besser an den jeweiligen Knochen angepasst werden. Es ist so ferner möglich, eine Verletzung der Weichteile durch hervorragende Teile der Fingergelenkprothese auszuschließen.

Abstrahiert betrifft die Erfindung eine Fingergelenkprothese mit einem Gelenkkörper, der ein erstes und ein zweites Gelenkelement aufweist, die schwenkbar um eine Drehachse miteinander verbunden sind und von denen sich jeweils eine Verankerungsleiste zur Befestigung an einem proximalen und/oder distalen Fingerglied erstreckt, wobei die Verankerungsleisten eine oder mehrere Befestigungsösen mit Öffnungen zur Aufnahme von Fixierungsschrauben aufweisen kann, und welche zur Vermeidung der zuvor genannten Nachteile von der Drehachse distal an der Verankerungsleiste von zumindest einem Gelenkelement ein endseitiges Befestigungselement aufweist.

Weitere Einzelheiten, Vorteile und Merkmale der Erfindung lassen sich dem nachfolgenden Teil der Beschreibung entnehmen, in dem ein Ausführungsbeispiel der erfindungsgemäßen Vorrichtung an Hand von 13 Zeichnungen näher erläutert ist. Es zeigen:
- Fig. 1: eine schematische radiale Ansicht eines Fingermittelgelenks, wobei der Ort für eine Implantation einer Fingergelenkprothese angedeutet ist;
- Fig. 2: eine perspektivische Ansicht der Fingergelenkprothese, wobei das endseitige Befestigungselement als Befestigungsöse an der distalen Verankerungsleiste ausgebildet ist;
- Fig. 3: eine perspektivische Ansicht der Fingergelenkprothese, wobei das endseitige Befestigungselement als Zylinder an der distalen Verankerungsleiste ausgebildet ist;
- Fig. 4: eine Draufsicht der Fingergelenkprothese mit einer Kombination von einem endseitigen Zylinder und einer endseitigen Befestigungsöse für die distale und proximale Verankerungsleiste;
- Fig. 5: eine Explosionsdarstellung des vorzugsweise zu verwendenden Scharniers;
- Fig. 6: eine perspektivische Ansicht der Fingergelenkprothese, wobei das endseitige Befestigungselement an der distalen als auch an der proximalen Verankerungsleiste als Befestigungsöse ausgebildet ist;
- Fig. 7: eine perspektivische Ansicht der Fingergelenkprothese gemäß Figur 6;
- Fig. 8: eine perspektivische Ansicht der Fingergelenkprothese gemäß Figur 6 mit acht Rippen, welche einen rechteckigen Querschnitt aufweisen;
- Fig. 9: eine perspektivische Ansicht der Fingergelenkprothese gemäß Figur 6 mit acht Rippen, welche einen dreieckigen Querschnitt aufweisen;
- Fig. 10: eine perspektivische Ansicht der Fingergelenkprothese gemäß Figur 6 mit acht Rippen, welche als Querschnitt aufweisen einen Halbkreis;
- Fig. 11: eine perspektivische Ansicht der Fingergelenkprothese mit ausschließlich zwei endseitigen Befestigungsösen;
- Fig. 12: eine perspektivische Ansicht der Fingergelenkprothese mit ausschließlichen lateralen, proximalen Befestigungsösen und einem endseitigen Befestigungselement, das in horizontaler Richtung und senkrecht zur Fingermittelachse als flügelartige Verbreiterung der distalen Verankerungsleiste vorgesehen ausgebildet ist;
- Fig. 13: eine perspektivische Ansicht der Fingergelenkprothese gemäß Figur 11 mit einer zusätzlichen lateralen, proximalen Befestigungsöse samt Ausnehmung an der proximalen Verankerungsleiste.

Gleiche oder gleich wirkende Bauteile sind mit denselben Bezugszeichen versehen.

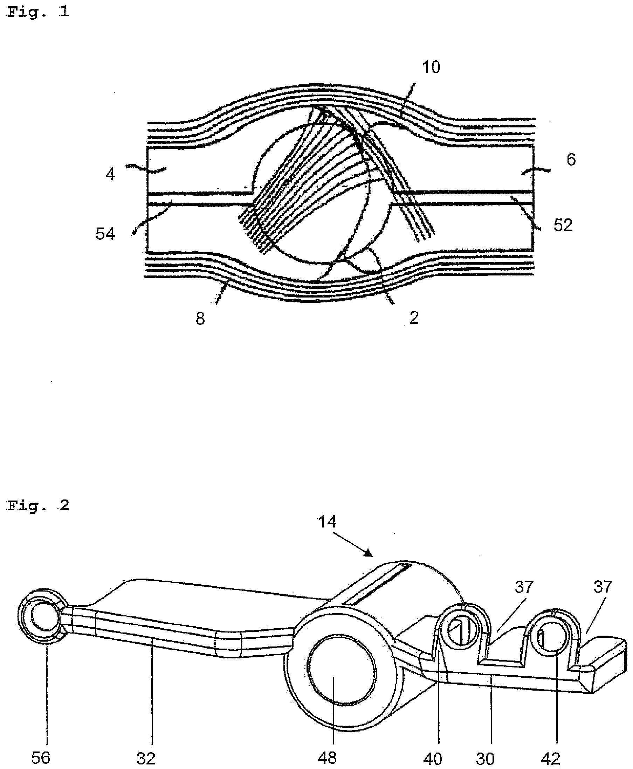

Figur 1 zeigt die radiale Seite eines Fingermittelgelenks 2 mit dem proximalen, d.h. körpernahen Fingerglied 4 und dem distalen, d.h. körperfernen Fingerglied 6. Unterhalb des Fingergliedes ist die Strecksehne 8 und oberhalb des Fingermittelgliedes die Beugesehne 10 dargestellt.

Die in den Figuren 2 - 5 gezeigte Fingergelenkprothese dient zur radialen Implantierung in das in Fig. 1 gezeigte Fingergelenk an dem Abschnitt des Scharniergelenks, der in Fig. 1 durch den Kreis in der Mitte angedeutet ist.

Figur 2 zeigt die zusammengesetzte Fingergelenkprothese in der perspektivischen Ansicht. Diese weist einen Gelenkkörper 14 auf, der ein erstes Gelenkelement mit einer proximalen Verankerungsleiste 30 und ein zweites Gelenkelement mit einer distalen Verankerungsleiste 32 umfasst, die so gelenkig miteinander verbunden sind, dass das zweite Gelenkelement bezüglich des ersten Gelenkelements um eine vertikale Achse 48 schwenkbar ist. Am Ende der distalen Verankerungsleiste 32 ist eine mediale Befestigungsöse 56 vorgesehen. Die proximalen Verankerungsleiste 30 weist laterale, proximale Befestigungsösen 40, 42, sowie entsprechende Ausnehmungen 37 auf.

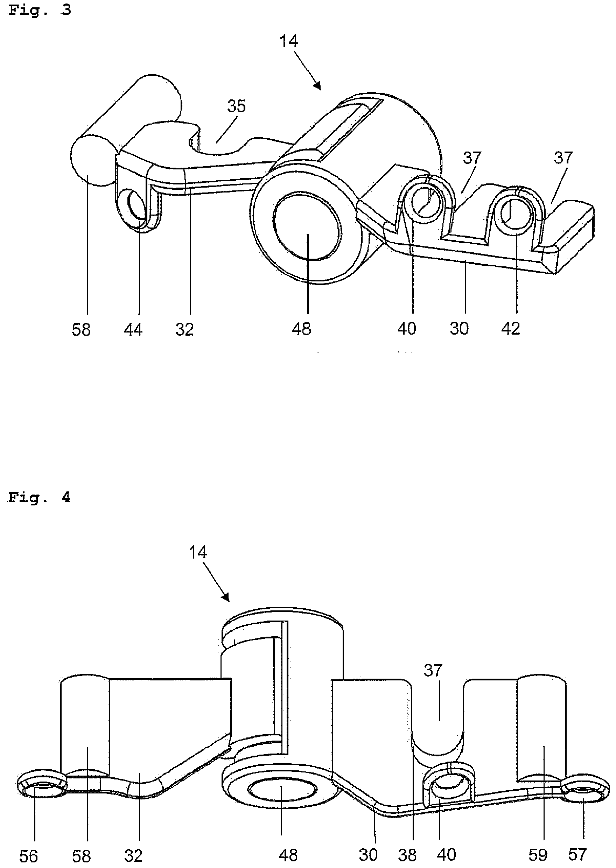

Figur 3 zeigt eine weitere Ausführung der zusammengesetzten Fingergelenkprothese in perspektivischer Ansicht. Das endseitige Befestigungselement ist hier als Zylinder 58 ausgebildet und befindet an derdistalen Verankerungsleiste 32. Diese weist ferner eine laterale, proximale Befestigungsöse 44 auf. Die Ausführung der proximalen Verankerungsleiste 30 entspricht derjenigen gemäß Figur 2.

Figur 4 zeigt in Draufsicht bzw. in Implantierungsstellung eine zusammengesetzte Fingergelenkprothese, wobei das erfindungsgemäße endseitige Befestigungselement an den von der Achse 48 entfernten Enden der distalen und proximalen Verankerungsleisten 32, 30 vorgesehen ist. Dabei wurden die in Fig. 2 und Fig. 3 dargestellten Ausführungen kombiniert. Die distale Verankerungsleiste 32 weist eine Befestigungsöse 56 und einen Zylinder 58 auf. Die proximale Verankerungsleiste 30 weist neben einer lateralen, proximalen Befestigungsöse 40 den Zylinder 59 und die Befestigungsöse 57 auf.

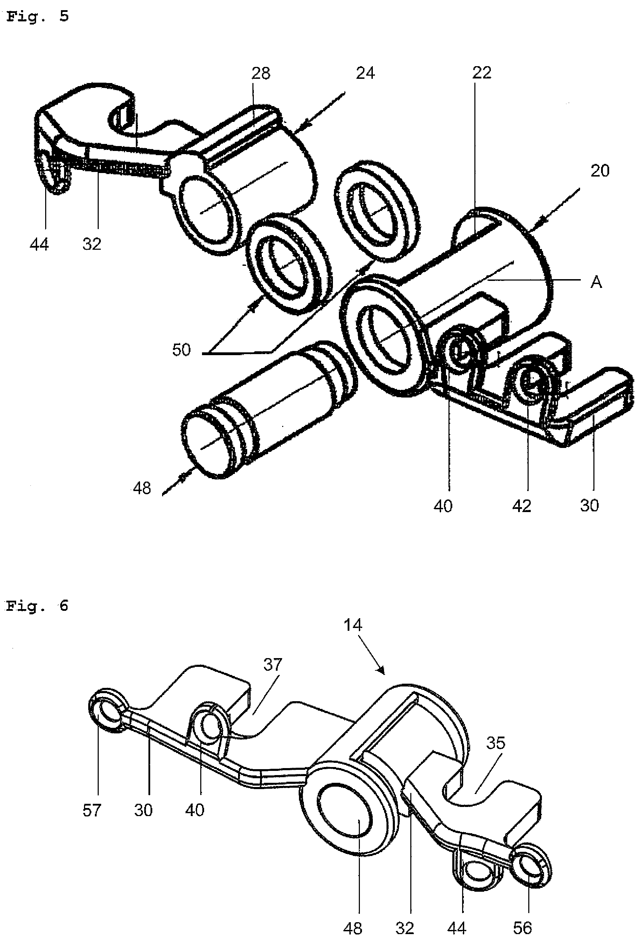

Figur 5 zeigt die Fingergelenkprothese mit dem vorzugsweise zu verwendenden Scharnier in Explosionsdarstellung. Das erste Gelenkelement umfasst einen Außenhohlzylinder 20, der einseitig von der Achse A eine Einsatzöffnung 22 aufweist. In diese sich über die Hälfte der Zylindermantelfläche erstreckende Einsatzöffnung 22 des Außenhohlzylinders 20 ist der Innenhohlzylinder 24 des zweiten Gelenkelements drehbeweglich einsetzbar. Außenseitig an dem Innenhohlzylinder 24 des zweiten Gelenkelements ist ein sich etwa über einen Winkelabschnitt von 90° erstreckender Anschlag 28 angeformt, welcher die Anschlagsränder des Innenhohlzylinders 24 bildet zum Anschlag an den Innenrändern der Einsatzöffnung 22 des Außenhohlzylinders 20. Durch entsprechende Ausbildung des Anschlags 28 kann der verstellbare Winkelbereich wunschgemäß realisiert bzw. eingeschränkt werden.

Figur 6 zeigt eine weitere Ausführung der zusammengesetzten Fingergelenkprothese in perspektivischer Ansicht. Das endseitige Befestigungselement ist als Befestigungsöse 57 ausgebildet und befindet sich am Ende der proximale Verankerungsleiste 30. Die proximale Verankerungsleiste 30 weist eine laterale, proximale Befestigungsöse 40 samt Ausnehmung 37 auf. Am Ende der distalen Verankerungsleiste 32 ist bei dieser Ausführung ebenfalls das endseitige Befestigungselement als Befestigungsöse 56 ausgebildet. Die distale Verankerungsleiste 32 weist ferner noch eine laterale, proximale Befestigungsöse 44 samt Ausnehmung 35 auf.

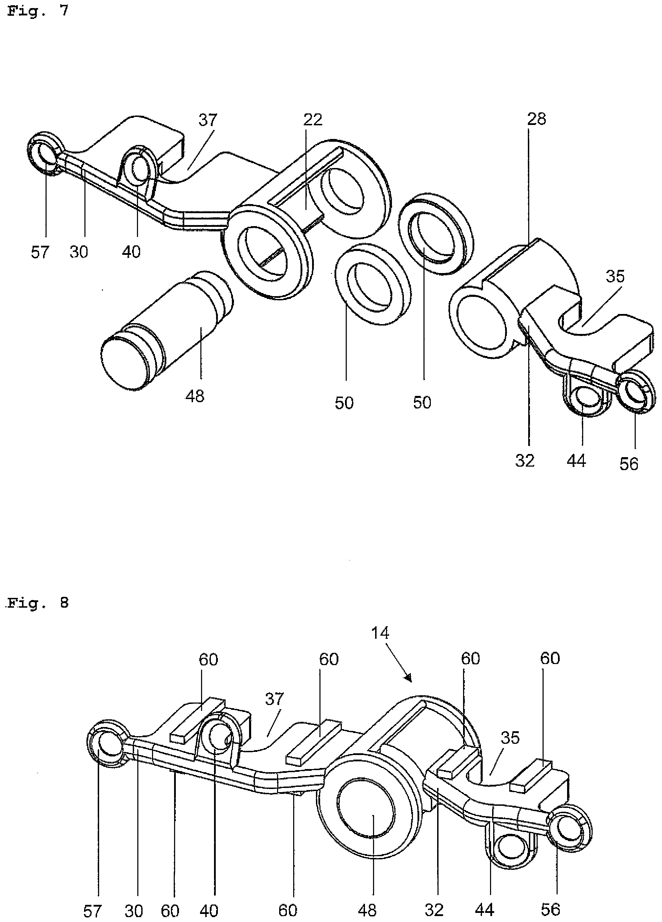

Figur 7 zeigt eine Explosionsdarstellung der Fingergelenkprothese gemäß Figur 6 mit einem Scharnier gemäß Figur 5.

Figur 8, 9 und 10 zeigten bevorzugte Ausführungsbeispiele einer Fingergelenkprothese gemäß Figur 6 mit 8 Rippen 60. Der Querschnitt ist dabei, rechteckig, dreieckig und als Halbkreis ausgebildet. Das Anwachsen und die Stabilität der Fingergelenkprothese werden durch die Rippen verbessert.

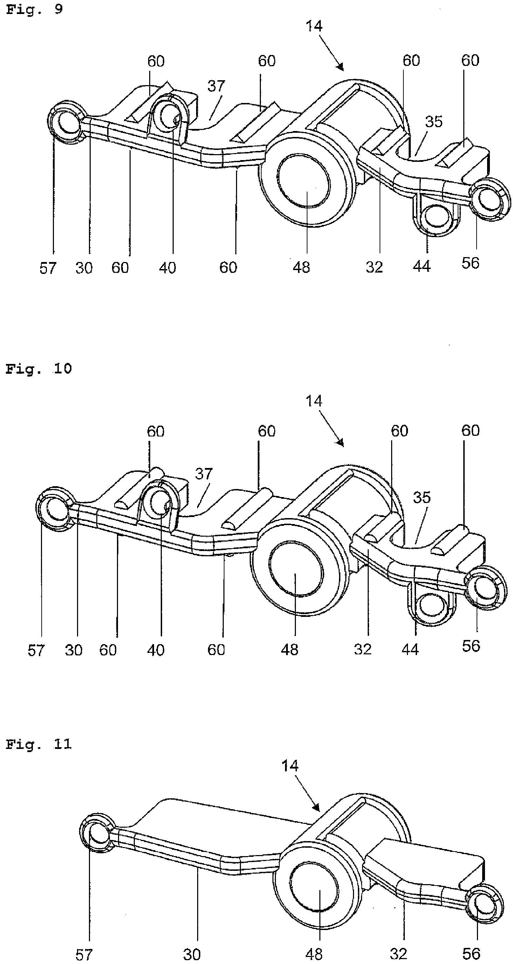

Figur 11 zeigt ein bevorzugtes Ausführungsbeispiel einer Fingergelenkprothese mit einem Scharnier gemäß Figur 5, jedoch mit ausschließlich zwei endseitigen Befestigungsösen. Wie dargestellt sind keine Ausnehmungen nötig oder vorgesehen.

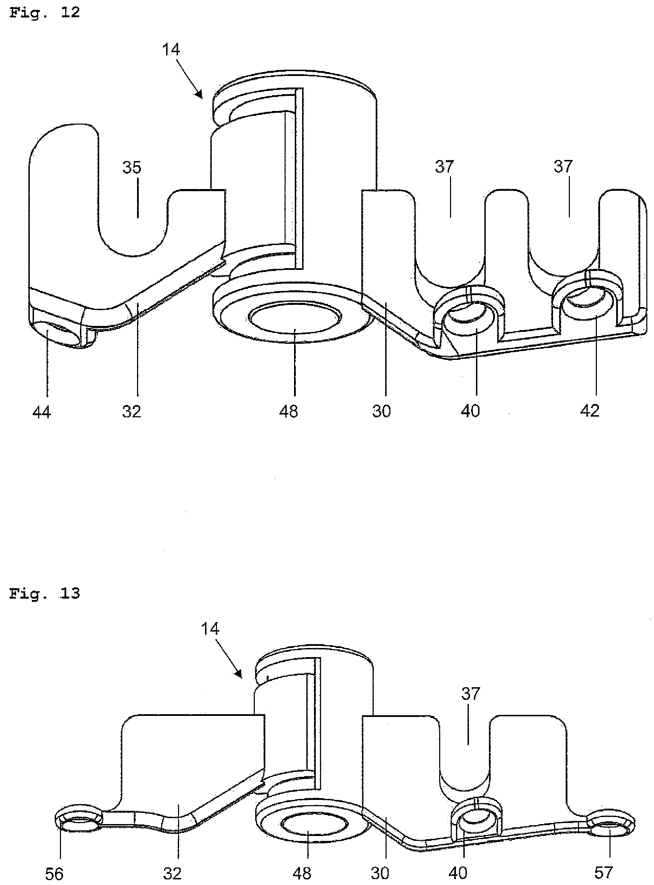

Figur 12 zeigt ein bevorzugtes Ausführungsbeispiel einer Fingergelenkprothese gemäß Figur 5 mit ausschließlich lateralen, proximalen Befestigungsösen und mit einem endseitigen Befestigungselement. Dieser ist in horizontaler Richtung und senkrecht zur Fingermittelachse als flügelartige Verbreiterung der distalen Verankerungsleiste ausgebildet und überragt im Einbau die Fingermittelachse. Dadurch reduziert das endseitige Befestigungselement ungünstige Hebelwirkungen. Es ist so möglich Ermüdungsbrüchen vorzubeugen. Das in den Knochen eingreifende Ende der flügelartigen Verbreiterung ist abgerundet. Frontseitig schließt die Verbreiterung bündig mit der Verankerungsleiste ab.

Figur 13 zeigt ein bevorzugtes Ausführungsbeispiel einer Fingergelenkprothese gemäß Figur 11, wobei an der proximalen Verankerungsleiste 30 eine laterale, proximale Befestigungsöse 40 samt entsprechender Ausnehmung 37 ausgebildet ist.

Einseitig an der Außenumfangsfläche des Außenhohlzylinders 20 ist eine sich proximal erstreckende, flächige Verankerungsleiste 30 einstückig angeformt. Diese proximale Verankerungsleiste 30 erstreckt sich von der Rückseite des Außenhohlzylinders 20 etwa bis zu dessen Mitte. Die proximale Verankerungsleiste 30 spannt eine Fläche auf, die sich quer zur Achse 48 des Gelenkkörpers 14 erstreckt. Die Höhe der proximalen Verankerungsleiste beträgt ca. ¼ der Gesamtlänge des Gelenkkörpers 14. Wie aus Figur 2 ersichtlich ist, ist die proximale Verankerungsleiste 30 etwas oberhalb einer durch den Achsmittelpunkt verlaufenden gedachten Horizontalebene an der Außenumfangsfläche des Außenhohlzylinders 20 angeformt.

Wie in der Figur 5 dargestellt, ist in einer Ebene mit der proximalen Verankerungsleiste 30 an dem Innenhohlzylinder 24 eine sich von der Rückseite bis etwa zur Mitte des Anschlags 28 ersteckende distale Verankerungsleiste 32 einstückig angeformt, die sich distal von dem Innenhohlzylinder 24 nach außen erstreckt und ebenfalls flächig ausgebildet ist.

Diese Verankerungsleisten 30, 32 sind deshalb flächig ausgebildet, um die in den Verankerungsleisten 30, 32 wirkenden Kräfte aufzunehmen.

Die distale Verankerungsleiste 32 kann aus der in den Figuren dargestellten Implantierstellung um ca. 90 Grad nach oben im Verhältnis zu der proximalen Verankerungsleiste 30 verschwenkt werden.

Bei der Verwendung von lateralen, proximalen Befestigungsösen 40, 42 und 44 sind diese so an der proximalen und distalen Verankerungsleiste 30, 32 angeordnet, dass diese im Bereich der Ausnehmungen 35, 37 derart gelegen sind, um auch ein schräges Eindrehen der Fixierungsschrauben in die Öffnungen der lateralen, proximalen Befestigungsösen 40, 42 und 44 zu ermöglichen. Um ein schräges Eindrehen der Befestigungsschrauben in die lateralen, proximalen Befestigungsösen 40, 42 und 44 besonders gut zu ermöglichen, sind an den Verankerungsleisten 30, 32 im Bereich der lateralen, proximalen Befestigungsösen 40, 42 und 44 an den Scheitelpunkten der Ausnehmungen 35, 37 Abschrägungen 38 ausgebildet. Die Materialstärke der Abschrägungen 38 nimmt in Richtung der lateralen Befestigungsösen zu und zwar bis zur vollen Materialstärke der Verankerungsleiste von vorzugsweise 0,7 bis 1,3 mm. Die lateralen, proximalen Befestigungsösen 40, 42 und 44 können ferner aus der Vertikalen um ca. 5 bis 10 Grad geneigt sein, um ein schräges Eindrehen der Befestigungsschrauben zu unterstützen. Durch die erfindungsgemäße Fingergelenkprothese kann auf laterale, proximale Befestigungsösen ganz oder teilweise verzichtet werden.

Die Rückseite (in Einbaulage vom Knochen abgewandt) der proximalen Verankerungsleiste 30 erstreckt sich von der Rückseite des Außenhohlzylinders 20 in einem Winkel von ca. 45° nach hinten bis zu einem Scheitelpunkt und steigt von diesem bis zum äußeren Freiende in einem flachen Winkel an. Die so gebildeten Schenkel der Verankerungsleisten 30, 32 schließen zwischen sich einen stumpfen Winkel ein, der vorzugsweise 150 bis 170 Grad beträgt, um die Verankerungsleisten so an die natürliche Geometrie des Fingerknochens anzupassen und die Stabilität durch Erhöhung der Auflagefläche im Knochen zu verbessern, ohne die Bewegungsfähigkeit des Gelenks einzuschränken.

Zur relativbeweglichen Verbindung des ersten Gelenkelements mit dem zweiten Gelenkelement wird durch eine vorderseitige Stirnöffnung in der Stirnseite des Außenhohlzylinders 20 eine Achse 48, vorzugsweise aus Polyethylen bestehend, eingeschoben, welche die zylindrische Öffnung des Innenhohlzylinders 24 durchdringt und diesen so in dem Außenhohlzylinder 20 fixiert. Durch Wahl einer geeigneten Presspassung zwischen der Achse 48 und dem Außenhohlzylinder 20 ist die Achse 48 unverlierbar mit dem Außenhohlzylinder 20 verbunden. Am vorderen Ende weist die Achse 48 einen in die vordere Stirnfläche des Außenhohlzylinders 20 einpressbaren Stift mit verringertem Durchmesser auf.

Zur Reduzierung der Reibung zwischen den Stirnaußenseiten des aus Titannitrit gefertigten Innenhohlzylinders 24 und den Stirninnenseiten des ebenfalls aus Titannitrit gefertigten Außenhohlzylinders 20 sind Ringschreiben 50, vorzugsweise aus Polyethylen ausgebildet, eingesetzt.

Für die Implantation der Fingergelenkprothese wird nach Ablösen des entsprechenden Seitenbandes mittels eines Rundfräsers von der radialen Seite her eine Bohrung an dem Ort gefräst, der durch den zentralen Kreis in Figur angedeutet ist. Der Bohrungsmittelpunkt entspricht dem Drehmittelpunkt des proximalen und distalen Fingerglieds 4, 6 zu Beginn der Verschwenkung des distalen Fingergliedes 6 aus der Streckstellung in die Beugestellung. Der Innendurchmesser und die Höhe der Bohrung entsprechen dem Außendurchmesser und der Höhe des Gelenkkörpers 14. Nach dem Fräsen der Bohrung werden mit einem Schlitzfräser zwei sich in Längsmittelrichtung der Fingerglieder von der gefrästen Bohrung aus erstreckende Schlitze 52, 54 von der radialen Seite aus eingefräst. Die Form der Schlitze 52, 54 entspricht dabei der Geometrie der Verankerungsleisten 30, 32.

Nach dem Fräsen der Bohrung und dem Fräsen der Schlitze 52, 54 wird die Fingergelenkprothese mit ihrem vorderen Ende in die gefräste Bohrung eingeführt, wobei die Verankerungsleisten 30, 32 in die Schlitze 52, 54 eingeschoben werden, bis die lateralen Befestigungsösen 40, 42, 44, oder je nach Ausführung, die medialen Befestigungsösen 56 und 57 an dem Fingerknochen anliegen. Sind Zylinder 58, 59 vorgesehen, müssen natürlich auch dem Durchmesser der Zylinder 58, 59 angepasste Bohrungen eingebracht werden. Anschließend werden die Verankerungsleisten 30, 32 an dem Knochenmaterial des entsprechenden proximalen bzw. distalen Fingerglieds 4, 6 befestigt. Dieses erfolgt durch Eindrehen von Titanschrauben je nach Ausführung durch die jeweiligen Befestigungsösen 40, 42, 44, 56 und 57 hindurch in das Knochenmaterial.

Die Bestandteile der Fingergelenkprothese sind vorzugsweise aus Metall, insbesondere aus Titan oder Titannitrit hergestellt. Dieses Material hat den Vorteil, dass durch das Einwachsen von Knochen in die poröse Oberfläche des Materials eine dauerhafte hohe Implantatstabilität erreicht wird. Es ist ferner denkbar, die Oberfläche des Materials mit einer biokompatiblen Beschichtung zu versehen, die das Einwachsen fördert. Besonders vorteilhaft ist z. B. Hydroxylapatit.

Zur Erzielung einer geeigneten Gleitpaarung zwischen der Achse und den Hohlkörpern, weist die Achse vorzugsweise ein Material mit einem von dem Material der Hohlkörper unterschiedlichen Härtegrad auf, die aber zusammen eine gute Gleitpaarung bilden. Bei Hohlkörpern aus Titan oder Titannitrit hat sich eine Achse aus Kunststoff als vorteilhaft erwiesen, insbesondere eine Achse aus ultrahochmolekularem Polyethylen (UHMWPE). Es kommen jedoch auch alle weiteren geeigneten Materialpaarungen mit entsprechenden Gleiteigenschaften in Betracht.

Abhängig von der Größe des zu ersetzenden Fingergelenks liegen die Dimensionen der Fingergelenkprothese bevorzugt in folgenden Bereichen:

| | |
|---|---|
| Durchmesser des Gelenkkörpers: | 6 bis 8 mm |
| Durchmesser der Achse: | 3 bis 4 mm |
| Länge der Verankerungsleisten: | 4 bis 8 mm |
| Dicke der Verankerungsleisten: | 0,7 bis 1,3 mm |

Die Erfindung wurde anhand des Ersatzes des Fingermittelgelenks durch die erfindungsgemäße Fingergelenkprothese beschrieben. In gleicher Weise kann die Fingergelenkprothese bei entsprechender Anpassung der Dimensionen unter Beibehaltung der Größenverhältnisse auch für den Ersatz der Endgelenke der Finger verwendet werden. Es ist denkbar die Prothese auch für weitere Gelenke auszubilden. Dazu ist es sinnvoll, dass alle Kanten der Prothese an den jeweiligen Knochen harmonisch anpassbar sind. Dabei es besonders zweckmäßig, wenn das endseitige Befestigungselement nicht medial oder lateral im Knochen übersteht. Besonders bevorzugt ragt das endseitige Befestigungselement 0,2 mm von der Ebene der Verankerungsleiste hervor.

Der Gegenstand der vorliegenden Erfindung ergibt sich nicht nur aus dem Gegenstand der einzelnen Ansprüche, sondern aus der Kombination der einzelnen Ansprüche untereinander. Alle in den Unterlagen - einschließlich der Zusammenfassung - offenbarten Angaben und Merkmale, insbesondere die in den Zeichnungen dargestellten räumlichen Ausbildungen, werden als erfindungswesentlich beansprucht, soweit sie einzeln oder in Kombination gegenüber dem Stand der Technik neu sind.

### Bezugszeichenliste

- A: Achse
- 2: Fingermittelgelenk
- 4: proximales Fingerglied
- 6: distales Fingerglied
- 8: Strecksehne
- 10: Beugesehne
- 14: Gelenkkörper
- 20: Außenhohlzylinder
- 22: Einsatzöffnung
- 24: Innenhohlzylinder
- 28: Anschlag
- 30: proximale Verankerungsleiste
- 32: distale Verankerungsleiste
- 35: Ausnehmung
- 37: Ausnehmung
- 38: Abschrägung
- 40: erste, laterale, proximale Befestigungsöse
- 42: zweite, laterale, proximale Befestigungsöse
- 44: dritte, laterale, proximale Befestigungsöse
- 48: Achse
- 50: Ringscheibe
- 52: Schlitz
- 54: Schlitz
- 56: mediale, distale Befestigungsöse
- 57: mediale, proximale Befestigungsöse
- 58: medialer, distaler Zylinder
- 59: medialer, proximaler Zylinder
- 60: Rippe

## Patentansprüche

1. Fingergelenkprothese mit einem Gelenkkörper (14), der ein erstes und ein zweites Gelenkelement aufweist, die schwenkbar um eine Drehachse miteinander verbunden sind und von denen sich jeweils eine Verankerungsleiste (30, 32) zur Befestigung an einem proximalen und/oder distalen Fingerglied erstreckt, wobei die Verankerungsleisten (30, 32) eine oder mehrere Befestigungsösen (40, 42, 44) mit Öffnungen zur Aufnahme von Fixierungsschrauben aufweisen kann, , **DADURCH GEKENNZEICHNET, dass** von der Drehachse distal an der Verankerungsleiste (30, 32) von zumindest einem Gelenkelement ein endseitiges Befestigungselement vorgesehen, welches medial in Bezug auf die horizontale Mittelebene der Verankerungsleiste (30, 32) angeordnet ist.

2. Fingergelenkprothese nach Anspruch 1, **DADURCH GEKENNZEICHNET, dass** das endseitige Befestigungselement in horizontaler Richtung, senkrecht zur Fingermittelachse als flügelartige Verbreiterung einer Verankerungsleiste (30, 32) ausgebildet ist.

3. Fingergelenkprothese nach einem der vorhergehenden Ansprüche, **DADURCH GEKENNZEICHNET, dass** das endseitige Befestigungselement als endseitige Befestigungsöse (56, 57) ausgebildet ist.

4. Fingergelenkprothese nach einem der vorhergehenden Ansprüche, **DADURCH GEKENNZEICHNET, dass** das endseitige Befestigungselement aus mindestens zwei benachbarten, Befestigungsösen ausgebildet ist.

5. Fingergelenkprothese nach einem der vorhergehenden Ansprüche, **DADURCH GEKENNZEICHNET, dass** das endseitige Befestigungselement als Zylinder (58, 59) ausgebildet ist.

6. Fingergelenkprothese nach einem der vorhergehenden Ansprüche, **DADURCH GEKENNZEICHNET, dass** das endseitige Befestigungselement sowohl als Zylinder (58, 59) als auch als Befestigungsöse (56, 57) ausgebildet ist.

7. Fingergelenkprothese nach einem der vorhergehenden Ansprüche, **DADURCH GEKENNZEICHNET, dass** das endseitige Befestigungselement als Kombination oder als Vielfaches einzelner oder mehrerer denkbarer Ausbildungen des Befestigungselements vorgesehen ist.

8. Fingergelenkprothese nach einem der vorhergehenden Ansprüche, **DADURCH GEKENNZEICHNET, dass** die Oberfläche rau ausgebildet ist.

9. Fingergelenkprothese nach einem der vorhergehenden Ansprüche, **DADURCH GEKENNZEICHNET, dass** zumindest an einer flächigen Seite der Verankerungsleisten (30, 32) zumindest eine Rippe (60) ausgebildet ist.

## Claims

1. Finger joint prosthesis with a joint body (14) having a first joint element and a second joint element which are connected to each other pivotably about a rotation axis and from each of which there extends a respective anchoring bar (30, 32) for securing to a proximal and/or distal phalanx, wherein the anchoring bars (30, 32) can have one or more securing eyes (40, 42, 44) with openings to receive fixing screws, **characterized in that** a terminal securing element is provided, distally from the rotation axis, on the anchoring bar (30, 32) of at least one joint element, which terminal securing element is arranged medially in relation to the horizontal central plane of the anchoring bar (30, 32).

2. Finger joint prosthesis according to Claim 1, **characterized in that** the terminal securing element is configured in a horizontal direction, perpendicular to the central axis of the finger, as a wing-like widening of an anchoring bar (30, 32).

3. Finger joint prosthesis according to one of the preceding claims, **characterized in that** the terminal securing element is configured as a terminal securing eye (56, 57).

4. Finger joint prosthesis according to one of the preceding claims, **characterized in that** the terminal securing element is formed from at least two adjacent securing eyes.

5. Finger joint prosthesis according to one of the preceding claims, **characterized in that** the terminal securing element is configured as a cylinder (58, 59).

6. Finger joint prosthesis according to one of the preceding claims, **characterized in that** the terminal securing element is configured both as a cylinder (58, 59) and also as a securing eye (56, 57).

7. Finger joint prosthesis according to one of the preceding claims, **characterized in that** the terminal securing element is provided as a combination or as a multiple of one or several conceivable configurations of the securing element.

8. Finger joint prosthesis according to one of the preceding claims, **characterized in that** the surface is rough.

9. Finger joint prosthesis according to one of the preceding claims, **characterized in that** at least one rib (60) is formed at least on a flat face of the anchoring bars (30, 32).

## Revendications

1. Prothèse d'articulation digitale, comprenant un corps d'articulation (14) qui présente un premier et un deuxième élément d'articulation qui sont connectés l'un à l'autre de manière à pouvoir pivoter autour d'un axe de rotation, et depuis lesquels s'étend à chaque fois une langue d'ancrage (30, 32) pour la fixation à une phalange proximale et/ou distale, les langues d'ancrage (30, 32) pouvant présenter un ou plusieurs oeillets de fixation (40, 42, 44) avec des ouvertures pour recevoir des vis de fixation, **caractérisée en ce qu'**un élément de fixation du côté de l'extrémité est prévu distalement depuis l'axe de rotation au niveau de la langue d'ancrage (30, 32) d'au moins un élément d'articulation, lequel élément de fixation est disposé médialement par rapport au plan médian horizontal de la langue d'ancrage (30, 32).

2. Prothèse d'articulation digitale selon la revendication 1, **caractérisée en ce que** l'élément de fixation du côté de l'extrémité est réalisé dans la direction horizontale, perpendiculairement à l'axe médian du doigt, sous forme d'élargissement en forme d'aile d'une langue d'ancrage (30, 32).

3. Prothèse d'articulation digitale selon l'une quelconque des revendications précédentes, **caractérisée en ce que** l'élément de fixation du côté de l'extrémité est réalisé sous forme d'oeillet de fixation du côté de l'extrémité (56, 57).

4. Prothèse d'articulation digitale selon l'une quelconque des revendications précédentes, **caractérisée en ce que** l'élément de fixation du côté de l'extrémité est réalisé à partir d'au moins deux oeillets de fixation adjacents.

5. Prothèse d'articulation digitale selon l'une quelconque des revendications précédentes, **caractérisée en ce que** l'élément de fixation du côté de l'extrémité est réalisé sous forme de cylindre (58, 59).

6. Prothèse d'articulation digitale selon l'une quelconque des revendications précédentes, **caractérisée en ce que** l'élément de fixation du côté de l'extrémité est réalisé à la fois sous forme de cylindre (58, 59) et également sous forme d'oeillet de fixation (56, 57).

7. Prothèse d'articulation digitale selon l'une quelconque des revendications précédentes, **caractérisée en ce que** l'élément de fixation du côté de l'extrémité est prévu sous forme de combinaisons ou sous forme de multiples de réalisations individuelles ou de plusieurs réalisations envisageables de l'élément de fixation.

8. Prothèse d'articulation digitale selon l'une quelconque des revendications précédentes, **caractérisée en ce que** la surface est réalisée sous forme brute.

9. Prothèse d'articulation digitale selon l'une quelconque des revendications précédentes, **caractérisée en ce qu'**au moins une arête (60) est réalisée au moins au niveau d'un côté plat des langues d'ancrage (30, 32).
